(19) Europäisches Patentamt
European Patent Office
Office européen des brevets

(11) Publication number: **0 270 349 B1**

(12) **EUROPEAN PATENT SPECIFICATION**

(45) Date of publication of patent specification: **04.08.93**

(51) Int. Cl.5: **C07C 211/13**, C07C 209/62, C07C 311/18, A61K 31/13

(21) Application number: **87310591.0**

(22) Date of filing: **01.12.87**

Divisional application 92203575.3 filed on 01/12/87.

# (54) Use of an anti-neoplastic spermine derivative in the preparation of a pharmaceutical composition.

(30) Priority: **02.12.86 US 936835**

(43) Date of publication of application:
**08.06.88 Bulletin 88/23**

(45) Publication of the grant of the patent:
**04.08.93 Bulletin 93/31**

(84) Designated Contracting States:
**AT BE CH DE ES FR GB GR IT LI LU NL SE**

(56) References cited:
**EP-A- 0 116 693**

**Journal of Medicinal Chemistry, vol. 16, no. 1, January 1973, pages 1-5, Washington, DC, US M. Israel et al.:"Synthesis and antitumor evaluation of presumed cytotoxic metabolites of spermine and N,N'-bis(3-aminopropyl)nonane-1,9-diamine"**

**Journal of Biochemistry, vol. 100, no. 1, July 1986, pages 123-130, Tokyo, JP, M. Takahashi et al.:"Thermophilic HB8 DNA ligase: effects of polyethylene glycols and polyamines on blunt-end ligation of DNA"**

**Cancer Research, vol. 45, May 1985, pages 2050-2057, Philadelphia, US; C.W. Porter et al.: "Biological properties of N4- and N1,N8-spermidine derivaives in cultured L1210 leukemia cells"**

**Cancer Research, vol. 47, June 1, 1987, pages 2821-2825, Philadelphia, US, C.W. Porter et al.: "Relative abilities of bis(ethyl) derivatives of putrescine, spermidine, and spermine to regulate polyamine biosynthesis and inhibit L1210 leukemia cell growth"**

(73) Proprietor: **THE UNIVERSITY OF FLORIDA**
**223 Grinter Hall**
**Gainesville, Florida 32611(US)**

(72) Inventor: **Bergeron, Raymond J.**
**6220 NW 56 Lane**
**Gainesville Florida 32601(US)**

(74) Representative: **Hedley, Nicholas James Matthew et al**
**Stephenson Harwood One, St. Paul's Churchyard**
**London EC4M 8SH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

## Description

This invention was made with U.S. Government support under Grant NCDDG-CA37606, awarded by the National Cancer Institute. The U.S. Government has certain rights in this invention.

### Field of the Invention

The present invention relates to anti-neoplastic pharmaceutical compositions.

### Prior Art

In recent years a great deal of attention has been focused on the polyamines, e.g., spermidine, norspermidine, homospermidine, 1,4-diaminobutane (putrescine), and spermine. These studies have been directed largely at the biological properties of the polyamines probably because of the role they play in proliferative processes. It was shown early on that the polyamine levels in dividing cells,e.g., cancer cells, are much higher than in resting cells. See Janne et al, A. Biochim. Biophys. Acta. 473, 241 (1978); Fillingame et al, Proc. Natl. Acad. Sci. U.S.A. 72:4042 (1975); Metcalf et al, J. Am. Chem. Soc. 100:2551 (1978); Flink et al, Nature (London) 253:62 (1975); and Pegg et al, Polyamine Metabolism and Function, Am. J. Cell. Physiol. 243:212-221 (1982).

Several lines of evidence indicate that polyamines, particularly spermidine, are required for cell proliferation: (i) they are found in greater amounts in growing than in non-growing tissues; (ii) prokaryotic and eukaryotic mutants deficient in polyamine biosynthesis are auxotrophic for polyamines; and (iii) inhibitors specific for polyamine biosynthesis also inhibit cell growth. Despite this evidence, the precise biological role of polyamines in cell proliferation is uncertain. It has been suggested that polyamines, by virtue of their charged nature under physiological conditions and their conformational flexibility, might serve to stabilize macromolecules such as nucleic acids by anion neutralization. See Dkystra et al, Science, 149:48 (1965); Russell et al, Polyamines as Biochemical Markers of Normal and Malignant Growth (Raven, New York, 1978); Hirschfield et al, J. Bacteriol. , 101:725 (1970); Morris et al, ibid, p. 731; Whitney et al, ibid, 134:214 (1978); Hafner et al, J. Biol. Chem., 254:12419 (1979); Cohn et al, J. Bacteriol. 134:200 (1978); Pohjatipelto et al, Nature (London), 293:475 (1981); Mamont et al, Biochem. Biophys. Res. Commun. 81:58 (1978); Bloomfield et al, Polyamines in Biology and Medicine (D.R. Morris and L.J.. Morton, Eds. - Dekker, New York, 1981) pp. 183-205; Gosule et al, Nature, 259:333 (1976); Gabbay et al, Ann. N.Y. Acad. Sci., 171:810 (1970); Suwalsky et al, J. Mol. Biol., 42:363 (1969) and Liquori et al, J. Mol. Biol., 24:113 (1968).

However, regardless of the reason for increased polyamine levels the phenomenon can be and has been exploited in chemotherapy. See Sjoerdsma et al, Butterworths Int. Med. Rev.: Clin. Pharmacol. Ther. 35:287 (1984); Israel et al, J. Med. Chem., 16:1 (1973); Morris et al, Polyamines in Biology and Medicine; Dekker, New York, p. 223 (1981) and Wang et al, Biochem. Biophys. Res. Commun., 94:85 (1980).

It is an object of the present invention to provide novel anti-neoplastic pharmaceutical compositions.

J. Biochemisty, 100 (1), pp. 123-130 (1986) discloses $N^1$, $N^4$-diethylspermine and its use in a DNA ligation process.

### SUMMARY OF THE INVENTION

The foregoing and other objects are realized by the present invention, one embodiment of which is a pharmaceutical composition comprising an anti-neoplastic effective amount of the compound, $N^1$,$N^4$-diethylspermine having the formula:

$$CH_3CH_2\text{-}N^1H\text{-}(CH_2)_3\text{-}N^2H\text{-}(CH_2)_4\text{-}N^3H\text{-}(CH_2)_3\text{-}N^4H\text{-}CH_2CH_3$$

or a pharmaceutically acceptable acid addition salt thereof and a pharmaceutically acceptable carrier therefor.

The present invention also provides $N^1$,$N^4$ diethyl spermine and pharmaceutically acceptable salts thereof for use in therapy.

The present invention also comprises the use of the compound, $N^1$,$N^4$-diethylspermine, having the formula:

$$CH_3CH_2\text{-}N^1H\text{-}(CH_2)_3\text{-}N^2H\text{-}(CH_2)_4\text{-}N^3H\text{-}(CH_2)_3\text{-}N^4H\text{-}CH_2CH_3$$

or a pharmaceutically acceptable acid addition salt thereof in the manufacture of a medicament for antineoplastic therapeutic treatment.

## DETAILED DESCRIPTION OF THE INVENTION

$N^1,N^4$-diethylspermine is active against malignant cells in vitro and in vivo in human and non-human animals.

The compound is preferably synthesized by first forming a sulfonamide of the polyamine at all of the amino nitrogens (1) to activate the primary amines for monoethylation, and (2) to protect any secondary nitrogens from ethylation. Suitable sulfonating agents include alkyl, aryl and arylalkyl sulfonating agents of the general structure $RSO_2X$ wherein R is alkyl, aryl or arylalkyl and X is a leaving group, e.g., $Cl^-$, $Br^-$, etc. The sulfonation is accomplished by reacting the polyamine with 1.0 equivalent of sulfonating agent per nitrogen in the presence of a base, e.g. tertiary amine or a hydroxide. The reaction is best accomplished using aqueous sodium hydroxide as the base and p-toluenesulfonyl chloride (TsCl) as the sulfonating agent in a biphasic solvent system consisting of an organic solvent, e.g. methylene chloride, and water. The sulfonating agent is added in methylene chloride to an aqueous solution of the amine and sodium hydroxide and the reaction proceeds according to the following equation, using spermine as the base compound:

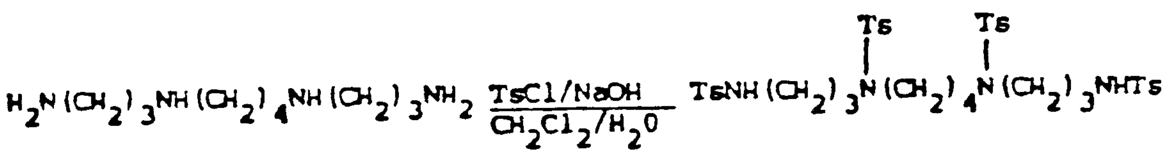


Wherein: Ts = p-toluenesulfonyl.

After purification the sulfonamide is next ethylated. The ethylation involves formation of N-anions on the primary amino sulfonamides with a base such as NaH followed by reaction of the N-anion with an ethylating agent $C_2H_5X$ wherein X is a leaving group such as $I^-$, $Cl^-$, $Br^-$, p-$CH_3C_6H_4SO_3^-$, $CH_3SO_3^-$.

The ethylation can be carried out in a variety of dipolar aprotic solvents, preferably, N, N-dimethylformamide (DMF). The reaction proceeds according to the following equation:

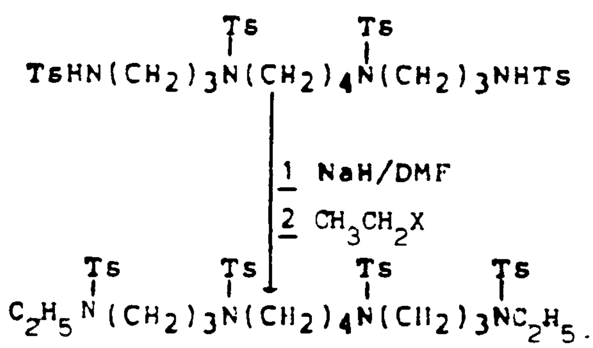


After ethylation of the sulfonamide, the sulfonyl protecting groups are next removed under reducing conditions. Although a variety of standard reducing conditions can be utilized ($LiAlH_4$, $Li/NH_3$, catalytic reduction), Na and $NH_3$ function optimally. The reduction proceeds according to the following equation:

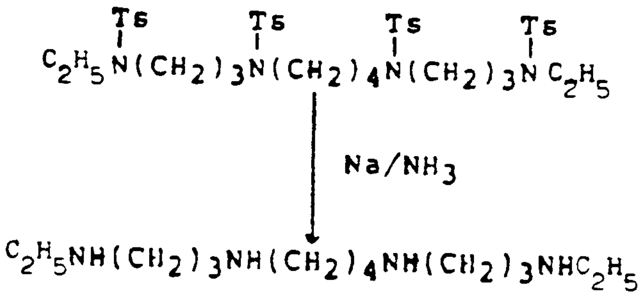


The compound is isolated as the free amine and then may be converted to and utilized as the corresponding hydrochloride salt by treatment with concentrated HCl. However, it may also be used as salts with any pharmaceutically acceptable acid, e.g., HBr, $CH_3CO_2H$, $CH_3SO_3H$, etc.

The invention is illustrated by the following non-limiting example.

## EXAMPLE

### Preparation of N[1],N[4]-diethylspermine

#### N[1],N[2],N[3],N[4]-Tetra-p-tosylspermine.

To spermine tetrahydrochloride (4.53 g, 13.0 mmol) and 10% aqueous NaOH (200 mL, 132 mmol) at 0° is added dropwise p-toluenesulfonyl chloride (9.98 g, 52.3 mmol) in $CH_2Cl_2$ with rapid stirring. After 1 hr the mixture is allowed to warm to room temperature and to stir for 2 days. The organic phase is separated and washed with 0.5 N HCl, $H_2O$, and brine, dried over $Na_2SO_4$ and purified on silica gel (450 g, 3% $MeOH/CHCl_3$) to give 9.69 g, 91% yield of tetratosylspermine.

NMR ($CDCl_3$) δ 7.2-7.9 (m, 16H), 5.34 (t, 2H, J=7), 2.9-3.3 (m, 12H), 2.43 (s, 12H), 1.5-2.0 (m, 8H).

N[1],N[4]-Diethyl-N[1],N[2],N[3],N[4]-Tetra-p-tosylspermine. To the tetratosylspermine prepared above (1.75 g, 2.14 mmol) in dry DMF 1(12 mL) was cautiously added 80% sodium hydride (0.25 g, 8.33 mmol) and then ethyl iodide (1.0 mL, 12.5 mmol). After heating under nitrogen (10 h, 55°), the mixture was quenched with ice water and extracted with chloroform (3 x). The organic phase was washed with 5% $Na_2SO_3$, 5% NaOH, 1N HCl, and water, then dried with $Na_2SO_4$. Removal of DMF by flash distillation and purification of the crude product on silica gel (4%$EtOH/CHCl_3$) produced 1.63 g (87%) of the desired product. NMR ($CDCl_3$) δ 7.2-7.8 (m, 16H), 3.0-3.3 (m, 16H), 2.43 (s, 12H), 1.5-2.1 (m, 8H), 1.08 (t, 6H, J=7). Anal. Calcd. for $C_{42}H_{58}N_4O_8S_4$; C, 57.64; H, 6.68; N, 6.40. Found: C, 57.69; H, 6.74; N, 6.20.

N[1],N[4]-diethylspermine (DES). Into a solution of the N[1],N[4]-diethyl-N[1],N[2],N[3],N[4]-tetratosylspermine prepared above (2.78 g, 3.18 mmoles) in dry, distilled THF (200 mL) at -78°C. was condensed 300 mL $NH_3$, using a dry ice condenser. Sodium spheres (3.0 g, 0.13 mol) were then added in small portions and the reaction mixture was stirred at -78°C. for 4 hr. The reaction mixture was allowed to warm to room temperature overnight and the $NH_3$ boiled off. Diethyl ether was added to the mixture. Ethanol was then cautiously added, then $H_2O$ was added to finally quench the reaction. The solvents were evaporated and the product extracted with diethyl ether and then chloroform. The extracts were dried over $Na_2SO_4$, filtered and the extracts concentrated. The resultant liquid was distilled in a Kugelrohr apparatus (150°C., 0.1 mm). Concentrated hydrochloric acid was added to an ether/ethanol (1:1) solution of the distillate to form the hydrochloride salt which was recrystallized from hot aqueous ethanol to give 790 mg (63%) DES. NMR ($D_2O$) δ 1.4 (t, 6H); 1.9 (m, 4H); 2.25 (m, 4H); 3.25 (m, 16H); 4.80 (s, HOD, reference).

The following protocols were followed to determine the $ID_{50}$ values for DES against cultured L1210 cells, Daudi cells and HL-60 cells.

#### Cell Culture.

Murine L1210 leukemia cells, human Burkitt lymphoma cells (Daudi) and human promyelocytic leukemia cells (HL-60) were maintained in logarithmic growth as suspension cultures in RPMI-1640 medium containing 2% 4-(1-hydroxyethyl)-1-piperazineethanesulfonic acid-3(N-morpholino)propanesulfonic acid, 100 µM aminoguanidine, and 10% fetal bovine serum. Cells were grown in 25 sq cm tissue culture flasks in a total volume of 10 mL under a humidified 5% $CO_2$ atmosphere at 37°C.

The cells were treated while in logarithmic growth (L1210 cells $0.3 \times 10^5$ cells/mL; Daudi and HL-60 $1 \times 10^5$ cells/mL) with the polyamine derivatives diluted in sterile water and filtered through a 0.2 micron filter immediately prior to use. Following a 48 h incubation with L1210 cells and a 72 h incubation with Daudi or HL-60 cells, L1210 cells were reseeded at $0.3 \times 10^5$ cells/mL, Daudi and HL-60 cells were reseeded at $1 \times 10^5$ cells/mL and all cells were incubated in the presence of the polyamine derivative for an additioal 48 h or 72 h.

Cell samples at the indicated time periods were removed for counting. Cell number was determined by electronic particle counting and confirmed periodically with hemocytometer measurements. Cell viability was assessed by trypan blue dye exclusion.

The percentage of control growth was determined as follows:

$$\% \text{ of control growth} = \frac{\text{Final treated cell no.} - \text{initial inoculum}}{\text{Final untreated cell no.} - \text{initial inoculum}} \times 100$$

The $IC_{50}$ is defined as the concentration of compound necessary to reduce cell growth to 50% of control growth.

The results are set forth in Tables 1 and 2.

TABLE 1. L1210 Cells

| | 48 H $IC_{50}$ | 96 H $IC_{50}$ |
|---|---|---|
| DES | 10 μM | 0.10 μM |

TABLE 2. Daudi Cells. HL-60 Cells

| | Daudi Cells 72 H $IC_{50}$ | Daudi Cells 144 H $IC_{50}$ | HL-60 Cells 72 H $IC_{50}$ | HL-60 Cells 144 H $IC_{50}$ |
|---|---|---|---|---|
| DES | > 40 μM | 0.5 μM | 10 μM | 0.3 μM |

Animal Studies.

The murine L1210 leukemia cells were maintained in DBA/2J mice. L1210 cells, from a single mouse which was injected i.p. with $10^6$ cells 5 days earlier, were harvested and diluted with cold saline so that there were $10^5$ or $10^6$ cells in 0.25 cc. For each study, mice were injected i.p. with $10^6$ L1210 cells or $10^5$ L1210 cells (See Table 3) on day O. The polyamine analogues were diluted in sterile saline within 24 h of use and the unused portion stored at 5 ° C.

DES was administered by i.p. injection 15 mg/kg or 20 mg/kg every 8 h for 3 days (days 1-3), 4 days (days 1-4), or 6 days (days 1-6) (see Table 3).

Mice which were treated with saline injections served as controls.

The parameter used for treatment evaluation was mean survival time.

(Percent increased life span, % ILS).

$$\% \text{ ILS} = \frac{\text{mean survival time treated animals} - \text{mean survival time controls}}{\text{mean survival controls}} \times 100$$

The murine Lewis lung carcinoma was maintained as s.c. tumor in C57B1/6 mice. The line was propagated every 14 days. A 2-4 mm fragment of s.c. donor tumor was implanted s.c. in the axillary region with a puncture in the inguinal region on day 0.

DES was administered by i.p. injection 20 mg/kg every 8 h for 5 days beginning on day 5 (days 5-9). Equal numbers of mice treated with saline injections served as controls. The parameter used for treatment evaluation was mean survival time (% ILS).

The parameters of the animal tests and results are set forth below in Tables 3 and 4.

TABLE 3

Evaluation of DES in DBA/2J
Male Mice With L1210 Leukemia (i.p.)

| DES Dosing Schedule | No. Animals | Day of Death | Mean Survival±SD | %ILS |
|---|---|---|---|---|
| 1)[a] 15mg/kg q 12hr days 1-6 | 6 | 14,14,14.5,15,15,17 | 14.9±1.3 | 55 |
| Control | 7 | 8.5,9.5,9.5,9.5,10,10,10 | 9.6±0.5 | 0 |
| 2)[b] 20mg/kg q8hr days 1-3 | 4 | 13.5,14,14,14.5 | 14.1±0.5 | 57 |
| Control | 4 | 8.5,8.5,9,10 | 9.0±0.5 | 0 |
| 3)[b] 20mg/kg q8hr days 1-4 | 10 | 14,14,15,15,16 17,18,20, 21,31[c] | 16.7±2.6 | 90 |
| Control | 9 | 8,8,9,9,9,9,9,9,10 | 8.8±0.4 | 0 |
| 4)[a] 15mg/kg q8hr days 1-6 | 8 | 8[c],20,22.5,24.5,28.5,33[d], 33[d],33[d] | 27.8±8.8 | 185 |
| Control | 6 | 8.5,9.5,10,10,10.5,10.5 | 9.8±0.7 | 0 |

[a]Mice injected with $10^5$ L1210 cells i.p. on day 0.
[b]Mice injected with $10^6$ L1210 cells i.p. on day 0.
[c]Death of animal not included in statistics...greater or less than Mean Survival ± 2x(S.D.).
[d]Experiment ended at 33 days. Animal survival evaluated on this day, however, these animals were alive with no sign of tumor.

TABLE 4

Evaluation of $N^1$, $N^4$-Di-ethylspermine (DES) in C57Bl/6J Male Mice with Lewis Lung Carcinoma (s.c.)

| Drug | Dose (mg/kg) | Schedule | Survival Values (Days) Mean ± S.D. | % ILS |
|---|---|---|---|---|
| DES | 20 (i.p.) | Every 8 h, days 5-9 | 43.7 ± 7.1 | 24 |
| Control (Saline) | - | - | 35.2 ± 2.6 | 0 |

The foregoing test results unequivocally establish the effectiveness of the composition of the invention as an anti-neoplastic agent.

DES may be compounded with any suitable pharmaceutically acceptable carrier such as phosphate buffered saline, saline, water, lactated ringers, dextrose (5% in water) and administered to a patient in need thereof intraveneously, intraperitoneally, subcutaneously, intramuscularly or orally,

Suitable dosages depend, of course, upon the nature of the malignant cells under treatment but generally range from about 1 to about 200 mg/kg per day.

**Claims**

**Claims for the following Contracting States : AT, BE, CH, DE, FR, GB, IT, LU, NL, SE, LI**

**1.** A pharmaceutical composition comprising an anti-neoplastic effective amount of the compound, $N^1$,$N^4$-diethylspermine having the formula:

$$CH_3CH_2\text{-}N^1H\text{-}(CH_2)_3\text{-}N^2H\text{-}(CH_2)_4\text{-}N^3H\text{-}(CH_2)_3\text{-}N^4H\text{-}CH_2CH_3$$

or a pharmaceutically acceptable acid addition salt thereof and a pharmaceutically acceptable carrier therefor.

**2.** Use of the compound, $N^1$, $N^4$-diethylspermine, having the formula:

$$CH_3CH_2\text{-}N^1H\text{-}(CH_2)_3\text{-}N^2H\text{-}(CH_2)_4\text{-}N^3H\text{-}(CH_2)_3\text{-}N^4H\text{-}CH_2CH_3.$$

or an acid addition salt thereof, in the manufacture of a medicament for antinecplastic therapy of a human or non-human animal body.

**3.** $N^1$,$N^4$-diethylspermine of the general formula:

$$CH_3CH_2\text{-}N^1H\text{-}(CH_2)_3\text{-}N^2H\text{-}(CH_2)_4\text{-}N^3H\text{-}(CH_2)_3\text{-}N^4H\text{-}CH_2CH_3$$

and pharmaceutically-acceptable salts thereof for use in therapy.

**Claims for the following Contracting States : ES, GR**

1. A process of making a pharmaceutical composition for use in anti-neoplastic therapy which comprises mixing an anti-neoplastic effective amount of $N^1$,$N^4$ diethylspermine

$$CH_3CH_2\text{-}N^1H\text{-}(CH_2)_3\text{-}N^2H\text{-}(CH_2)_4\text{-}N^3H\text{-}(CH_2)_3\text{-}N^4H\text{-}CH_2CH_3.$$

or a pharmaceutically acceptable acid addition salt thereof with a pharmaceutically acceptable carrier therefor.

2. Use of the compound, $N^1$, $N^4$-diethylspermine, having the formula:

$$CH_3CH_2\text{-}N^1H\text{-}(CH_2)_3\text{-}N^2H\text{-}(CH_2)_4\text{-}N^3H\text{-}(CH_2)_3\text{-}N^4H\text{-}CH_2CH_3.$$

or an acid addition salt thereof, in the manufacture of a medicament for antineoplastic therapy of a human or non-human animal body.

**Patentansprüche**
**Patentansprüche für folgende Vertragsstaaten : AT, BE, CH, DE, FR, GB, IT, LU, NL, SE, LI**

1. Ein pharmazeutisches Gemisch, das eine anti-neoplastisch wirksame Menge der Verbindung $N^1$, $N^4$-Diethylspermin enthält mit der Formel:

$$CH_3CH_2\text{-}N^1H\text{-}(CH_2)_3\text{-}N^2H\text{-}(CH_2)_4\text{-}N^3H\text{-}(CH_2)_3\text{-}N^4H\text{-}CH_2CH_3$$

oder ein pharmazeutisch akzeptables, durch Säurezugabe gewonnenes Salz hiervon und einen pharmazeutisch akzeptablen Träger hierfür.

2. Anwendung der Verbindung $N^1$, $N^4$-Diethylspermin mit der Formel:

$$CH_3CH_2\text{-}N^1H\text{-}(CH_2)_3\text{-}N^2H\text{-}(CH_2)_4\text{-}N^3H\text{-}(CH_2)_3\text{-}N^4H\text{-}CH_2CH_3$$

oder eines durch Säurezugabe gewonnenen Salzes hiervon für die Herstellung eines Medikamentes zur anti-neoplastischen Therapie bei einem menschlichen oder nichtmenschlichen tierischen Körper.

3. $N^1$, $N^4$-Diethylspermin der allgemeinen Formel:

$$CH_3CH_2\text{-}N^1H\text{-}(CH_2)_3\text{-}N^2H\text{-}(CH_2)_4\text{-}N^3H\text{-}(CH_2)_3\text{-}N^4H\text{-}CH_2CH_3$$

und pharmazeutisch akzeptable Salze hiervon für den Therapiegebrauch.

**Patentansprüche für folgende Vertragsstaaten : ES, GR**

1. Verfahren zur Herstellung einer pharmazeutischen Zusammensetzung zum Gebrauch in einer anti-neoplastischen Therapie, das enthält: Mischen einer anti-neoplastisch wirksamen Menge $N^1$, $N^4$-Diethylspermin

$$CH_3CH_2\text{-}N^1H\text{-}(CH_2)_3\text{-}N^2H\text{-}(CH_2)_4\text{-}N^3H\text{-}(CH_2)_3\text{-}N^4H\text{-}CH_2CH_3$$

oder eines pharmazeutisch akzeptablen durch Säurezugabe gewonnenen Salzes hiervon mit einem pharmazeutisch aktzeptablen Träger hierfür.

2. Anwendung der Verbindung $N^1$, $N^4$-Diethylspermin mit der Formel:

$$CH_3CH_2\text{-}N^1H\text{-}(CH_2)_3\text{-}N^2H\text{-}(CH_2)_4\text{-}N^3H\text{-}(CH_2)_3\text{-}N^4H\text{-}CH_2CH_3$$

oder eines durch Säurezugabe gewonnenen Salzes hiervon bei der Herstellung eines Medikamentes zur anti-neoplastischen Therapie bei einem menschlichen oder nichtmenschlichen tierischen Körper.

**Revendications**
**Revendications pour les Etats contractants : AT, BE, CH, DE, FR, GB, IT, LI, LU, NL, SE**

1. Composition pharmaceutique comprenant une quantité efficace antinéoplastique du composé $N^1,N^4$-diéthylspermine de formule :

$CH_3CH_2\text{-}N^1H\text{-}(CH_2)_3\text{-}N^2H\text{-}(CH_2)_4\text{-}N^3H\text{-}(CH_2)_3\text{-}N^4H\text{-}CH_2CH_3$

ou d'un sel d'addition acide pharmaceutiquement acceptable d'un tel composé et un véhicule pharmaceutiquement acceptable pour un tel composé.

2. Utilisation du composé $N^1,N^4$-diéthylspermine de formule :

$CH_3CH_2\text{-}N^1H\text{-}(CH_2)_3\text{-}N^2H\text{-}(CH_2)_4\text{-}N^3H\text{-}(CH_2)_3\text{-}N^4H\text{-}CH_2CH_3$

ou d'un sel d'addition acide d'un tel composé, dans la fabrication d'un médicament pour la thérapie antinéoplastique du corps humain ou animal autre qu'humain.

3. $N^1,N^4$-diéthylspermine de formule générale :

$CH_3CH_2\text{-}N^1H\text{-}(CH_2)_3\text{-}N^2H\text{-}(CH_2)_4\text{-}N^3H\text{-}(CH_2)_3\text{-}N^4H\text{-}CH_2CH_3$

et sels pharmaceutiquement acceptables d'un tel composé pour une utilisation en thérapie.

**Revendications pour les Etats contractants : ES, GR**

1. Procédé de fabrication d'une composition pharmaceutique pour l'utilisation en thérapie antinéoplastique qui consiste à mélanger une quantité efficace antinéoplastique de $N^1,N^4$-diéthylspermine de formule :

$CH_3CH_2\text{-}N^1H\text{-}(CH_2)_3\text{-}N^2H\text{-}(CH_2)_4\text{-}N^3H\text{-}(CH_2)_3\text{-}N^4H\text{-}CH_2CH_3$

ou d'un sel d'addition acide pharmaceutiquement acceptable d'un tel composé avec un véhicule pharmaceutiquement acceptable pour un tel composé.

2. Utilisation du composé $N^1,N^4$-diéthylspermine de formule :

$CH_3CH_2\text{-}N^1H\text{-}(CH_2)_3\text{-}N^2H\text{-}(CH_2)_4\text{-}N^3H\text{-}(CH_2)_3\text{-}N^4H\text{-}CH_2CH_3$

ou d'un sel d'addition acide d'un tel composé, dans la fabrication d'un médicament pour la thérapie antinéoplastique du corps humain ou animal autre qu'humain.